(19) Europäisches Patentamt
European Patent Office
Office européen des brevets

(11) Publication number: **0 152 757 B1**

(12) **EUROPEAN PATENT SPECIFICATION**

(45) Date of publication of patent specification: **19.10.88**

(51) Int. Cl.$^4$: **C 07 C 29/10,** C 07 C 35/18

(21) Application number: **85100323.6**

(22) Date of filing: **15.01.85**

(54) **Preparation of terpinen-4-ol.**

(30) Priority: **16.01.84 US 570799**

(43) Date of publication of application:
**28.08.85 Bulletin 85/35**

(45) Publication of the grant of the patent:
**19.10.88 Bulletin 88/42**

(84) Designated Contracting States:
**DE FR GB IT NL**

(56) References cited:
**PERFUMER ESSENT. OILS REC., November 1966, pages 694-698; J. VERGHESE: "Terpinen-4-Ol"**
**CHEMICAL ABSTRACTS, vol. 88, 1978, page 626, no. 152783n, Columbus, Ohio, US; Y. BESSIERE et al.: "Isomerization of limonene epoxides. Allylic rearrangement of p-mentha-1(7),8-dien-2-ols: preparation of perilla alcohol"**
**SYNTHESIS, no. 6, June 1980, pages 492-493, Georg Thieme Verlag, Stuttgart, DE; T. KOBAYASHI et al.: "Reductive cleavage of 5,6-dihydro-2H-pyran derivatives; facile synthesis of cis-3-hexenol"**

(73) Proprietor: **Union Camp Corporation**
**1600 Valley Road**
**Wayne New Jersey 07470 (US)**

(72) Inventor: **Mitchell, Peter W.D.**
**106 Lancaster Road**
**Freehold, NJ 07470 (US)**

(74) Representative: **von Füner, Alexander, Dr. et al**
**Patentanwälte v. Füner, Ebbinghaus, Finck**
**Mariahilfplatz 2 & 3**
**D-8000 München 90 (DE)**

**EP 0 152 757 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

Field of the Invention

The invention relates to a method for the preparation of terpinen-4-ol and intermediates thereof.

SUMMARY OF THE INVENTION

The invention comprises a method of preparing terpinen-4-ol, which comprises; E2 elimination of 1,4-cineole.

DETAILED DESCRIPTION OF THE PREFERRED EMBODIMENTS OF THE INVENTION

Terpinen-4-ol is a fragrance chemical possessing a pleasing earthy-green note with a slightly peppery-woody undernote. It is an important constituent of synthetic essential oils, especially geranium, lavender and rose oils and has the desirable effect in all fragrance compositions of enhancing naturalness and diffusiveness. Because of the high cost and uncertainty of supply of the natural product, isolated from tea tree oil, synthetic routes to terpinen-4-ol have been developed, notably via photo-oxidation (Tetrahedron 21 (8), 2173—8 (1965)) or epoxidation of terpinolene (US—A 4 600 800 and 4 496 776). Both of these processes suffer from only modest overall yields and multiple chemical steps. The photochemical route requires expensive specialized equipment.

The procedure of the present invention has two major advantages over the prior art. First, it is a single step process. Second, it proceeds in very high selectivity. This procedure has the further advantage that it uses as its starting material a low-valued by-product, 1,4-cineole, generated during the production of pine oil by hydration of the abundant turpentine component, *alpha*-pinene. In "Perfumer and Essential Oil Record", *57*, 694—698 (November 1966) it is described that terpinen-4-ol was obtained from essential oils by fractionation as no crystalline derivative of the alcohol was known from which it can be generated. It is reported that an elegant method was announced for extracting terpinen-4-ol through its borate; the parent alcohol is released from the borate by boiling with 50% caustic potash or 60% acetic acid.

Refinements in the separation of terpinen-4-ol from oils involve GLC fractionation, a combination of (a) precise and GLC fractionation and (b) precise column and GLC fractionation.

It is further reported that terpinen-4-ol can be prepared from (1) alpha-Terpinen derivatives (2) Terpinolene and (3) alpha-Thujene and Sabinene.

In "Synthesis", No. 6, June 1980, pages 492—493 it is reported a novel two-step synthesis of cis-3-hexenol from commercially available 1,3-pentadiene and formaldehyde. 1,3-Pentadiene (1) and paraformaldehyde (2) were heated at 240°C to give 2-methyl-5,6-dihydr-2H-pyran (3) in 26% yield. The Diels-Alder reaction was regiospecific in the expected isomer and the 3-methyl-3,6-dihydro-2H-pyran was not detected.

Reductive cleavage of the allylic ether linkage in the compound was carried out by lithium metal in ethylamine at −78°C. The hexenol fraction (91%) was found by GLC and 'H—NMR analyses to be a mixture of 4,trans-3-hexenol, and trans-4-hexenol in a ratio of 92:1:7. In performing the reductive cleavage using sodium or lithium in other solvents such as liquid ammonia, diethylamine, and ethylenediamine at various temperatures, both the selectivity and yield were decreased. Although the cleavage reaction of cyclic ethers has been studied, such a simple and selective cleavage reaction is unprecedented and several experiments were carried out for other 5,6-dihydro-2H-pyran derivatives.

The method of the invention is carried out by displacement of the ether bridge on the 1,4-cineole molecule. The reaction, an E2 elimination, may be effected by reacting the 1,4-cineole with an elimination reagent. Representative of elimination reagents are the alkali metal salts of ammonia and aliphatic amines and diamines. The solvent employed is an amine or diamine solvent for the salt. Preferred solvents are diamines having the formula:

$$RNH{-}(CH_2)_n{-}HNR' \qquad (I)$$

wherein R and R' are each selected from the group consisting of hydrogen, alkyl and aminoalkyl and n is an integer of 1, 2, 3 or 4. The term "alkyl" as used herein means the monovalent moiety obtained upon removal of a hydrogen atom from a parent alkane. Representative of alkyl are alkyl of 1 to 25 carbon atoms, inclusive, such as methyl, ethyl, propyl, butyl, pentyl, hexyl, heptyl, octyl, nonyl, undecyl, decyl, dodecyl, octadecyl, nonadecyl, eicosyl, heneicosyl, docosyl, tricosyl, tetracosyl, pentacosyl and the isomeric forms thereof. Especially preferred are alkyl rests with 1 to 6 carbon atoms.

The term "aminoalkyl" as used herein means alkyl as defined above wherein a hydrogen atom has been replaced by an amino group. Representative of aminoalkyl are aminomethyl, aminoethyl, aminopropyl, aminobutyl, aminooctadecyl, aminotricosyl, aminopentacosyl and the like.

Preferred as elimination reagents used in the method of the invention are the lithium, sodium or potassium salts of ammonia, ethylenediamine, 1,3-diaminopropane and 3,3'-diamino-N-methyldipropylamine.

The method of the invention may be carried out by simple admixture of the 1,4-cineole with the elimination reagent. The reaction may be represented schematically by the formulae:

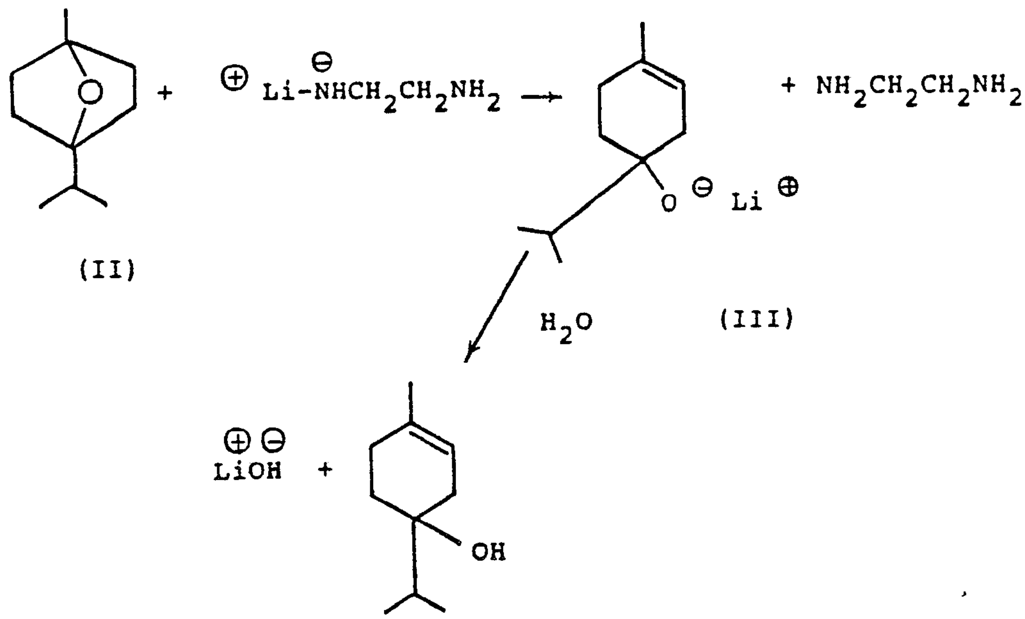

(II)

(III)

(IV)

wherein N-lithioethylenediamine is depicted as illustrative of the elimination reagents. Addition of water to the reaction mixture converts the intermediate lithium terpinenate of formula (III) to the free alcohol, IV (terpinen-4-ol).

Although temperature or pressure is not critical, the above-described reaction proceeds advantageously at a temperature within the range of from about 50 to 200°C, preferably 90—180°C, most preferably 100—140°C.

The proportions of reactants employed in the method of the invention are also not critical, stoichiometric proportions being acceptable. Preferably a small excess of the elimination reagent is employed.

In a preferred embodiment method of the invention, lithium salts of the diamine are formed in-situ in the reaction mixture by addition of the lithium metal and the amine of formula (I) independently to the reaction mixture charge. In this case, the molar proportion of reactants charged to the reaction mixture is advantageously within the range of 2—4:1—2:1—2 (amine:lithium:cineole), most preferably 3:1:1.

The reaction is conveniently carried out in conventional reaction apparatus. Progress of the reaction may be followed employing known analytical technique. In general the reaction is complete within 1/2 to 12 hours. Upon termination of the reaction, the desired terpinen-4-ol may be separated from the reaction mixture by conventional techniques such as by distillation.

Example 1

Lithium wire (21,6 g, 3,08 mole), 1,3-diaminopropane (683 g, 9.2 mole), and technical grade 1,4-cineole (72%, 504 g, 2.4 mole) were charged to a 2-liter Morton flask equipped with stirrer, condenser, thermometer, and nitrogen inlet. This mixture was heated with stirring to 110°C and held at this temperature for 9 hours. The reaction mixture was then allowed to cool to about 50°C. Water (55 g, 3.1 mole) was added, and the mixture then stirred at 80°C for 30 minutes. The product mass was then transferred to a distillation flask and the bulk of the amine and some terpene hydrocarbons were removed through a 5-plate Oldershaw column at 20 mm Hg pressure. The residue was washed with water to yield 387 g of crude terpinen-4-ol containing 80.6% true terpinen-4-ol. This represents a yield of 88%.

Example 2

Ethylenediamine (3.6 g, 0.060 mole) was weighed into a 50 ml three neck round bottom flask equipped with a condenser, thermometer, and nitrogen inlet. With stirring, lithium pieces (1.2 g) were added to the ethylene diamine. The mixture was heated to 110°C. Once the color of the solution turned a bluish-black, 3.1 gram (0.020 mole) of 1,4-cineole (91.3% 1,4-cineole, 7.7% 1,8-cineole) was added to the solution. The mixture was heated at 110°C for 4 hours. The final products was recovered by diluting the mixture with 10 ml of water, adding 10 ml of methylene chloride, stirring for 10 minutes, and then separating the layers. Gas chromatographic analysis of the product showed a 55.5% 1,4-cineole, 7.4% 1,8-cineole, and 33.1% terpinen-4-ol representing a molar selectivity of 92% to terpinen-4-ol on a conversion of 39%.

Example 3

The procedure of Example 2 *supra.* was followed using 3,3'diamino-N-methyldipropylamine in place of ethylenediamine. The reaction temperature was raised gradually during the run from 110°C to 180°C. Analysis of the product showed 44.5% 1,4-cineole, 6.3% 1,8-cineole and 38.2% terpinen-4-ol representing a molar selectivity of 79% to terpinen-4-ol on a conversion of 49%.

Example 4

Ethylenediamine (25 ml) and 0.016 mole of 1,4-cineole were weighed into a 50 ml round bottom flask. The flask was placed in a nitrogen atmosphere and 0.91 grams (0.023 mole) of sodium amide added. The mixture was heated at 110°C for 1 hour. The product was recovered by diluting the mixture with 20 ml of water and decanting the oil. Weight percent gas chromatographic analysis of the product showed 14.2% 1,4-cineole, 6.8% 1,8-cineole and 72.2% terpinen-4-ol representing a molar selectivity of 96.3% to terpinen-4-ol on a conversion of 84.1%.

**Claims**

1. A method of preparing terpinen-4-ol, which comprises; an E2 elimination of 1,4-cineole.

2. A method of preparing terpinen-4-ol, which comprises: reacting 1,4-cineole with a compound selected from the group consisting of an alkali metal salt of ammonia, an alkylamine and an alkyl diamine in an amine or diamine solvent of the formula:

$$RNH-(CH_2)_n-HNR'$$

wherein R and R' are each selected from the group consisting of hydrogen, alkyl and aminoalkyl and n is an integer of 2 or 3; and hydrolyzing the reaction product.

3. The method of claim 2 wherein the elimination is carried out at a temperature within the range of from about 50° to about 200°C.

4. The method of claim 3 wherein the temperature is within the range of from about 100°C to 140°C.

5. The method of one of the claims 2 to 4 wherein the diamine is selected from the group consisting of ethylenediamine, 1,3-diaminopropane and 3,3'-diamino-N-methyldipropylamine.

6. The method of one of the claims 2 to 4 wherein the lithium salt is formed in-situ in the reaction mixture by reaction of lithium metal with the diamine.

7. The method of one of the claims 2 to 4 wherein the elimination agent is sodium amide.

**Patentansprüche**

1. Verfahren zur Herstellung von Terpinen-4-ol, das die E2-Abspaltung von 1,4-Cineol umfaßt.

2. Verfahren zur Herstellung von Terpinen-4-ol, das die Umsetzung von 1,4-Cineol mit einer Verbindung, ausgewählt aus der Gruppe bestehend aus einem Alkalimetallsalz von Ammoniak, einem Alkylamin und einem Alkyldiamin, in einem Amin- oder Diaminlösungsmittel der Formel

$$RNH-(CH_2)_n-HNR',$$

worin R und R' jeweils ausgewählt sind aus der Gruppe, bestehend aus Wasserstoff, Alkyl und Aminoalkyl, und n eine ganze Zahl von 2 oder 3 bedeutet, und das Hydrolisieren des Reaktionsprodukts umfaßt.

3. Verfahren nach Anspruch 2, bei dem die Abspaltung bei einer Temperatur im Bereich von ungefähr 50°C bis ungefähr 200°C durchgeführt wird.

4. Verfahren nach Anspruch 3, bei dem die Temperatur im Bereich von ungefähr 100°C bis ungefähr 140°C liegt.

5. Verfahren nach einem der Ansprüche 2 bis 4, bei dem das Diamin ausgewählt ist aus der Gruppe, bestehend aus Ethylendiamin, 1,3-Diaminpropan und 3,3'-Diamino-N-methyldipropylamin.

6. Verfahren nach einem der Ansprüche 2 bis 4, bei dem das Lithiumsalz in-situ im Reaktionsgemsich durch Umsetzung des Lithiummetalls mit dem Diamin hergestellt wird.

7. Verfahren nach einem der Ansprüche 2 bis 4, bei dem das Abspaltungsagens Natriumamid ist.

**Revendications**

1. Un procédé de préparation du terpinen-4-ol consistant en une élimination E2 de 1,4-cinéole.

2. Un procédé de préparation du terpinen-4-ol qui consiste à faire réagir le 1,4-cinéole avec un dérivé choisi dans le groupe constitué par les sels de métal alcalin d'ammonium, l'alkylamine et les alkyl diamines dans une amine ou une diamine servant de solvant, présentant la formule:

$$RNH-(CH_2)_n-HNR'$$

dans laquelle: R et R' sont tous deux choisis dans le groupe constitué par un atome d'hydrogène, un radical

alkyle ou amino-alkyle; et n représente un entier égal à 2 ou 3; et à hydrolyser le produit de la réaction.

3. Le procédé selon la revendication 2, dans lequel l'élimination est mise en oeuvre à une température comprise entre environ 50°C et 200°C.

4. Le procédé selon la revendication 3, dans lequel la température est comprise entre environ 100°C et 140°C.

5. Le procédé selon l'une quelconque des revendications 2 à 4, dans lequel la diamine est choisie dans le groupe constitué par l'éthylènediamine, le 1,3-diaminopropane et la 3,3'-diamino-N-méthyldipropylamine.

6. Le procédé selon l'une quelconque des revendications 2 à 4, dans lequel le sel de lithium est formé in-situ dans le mélange réactionnel par réaction du lithium métallique avec la diamine.

7. Le procédé selon l'une quelconque des revendications 2 à 4, dans lequel l'agent d'élimination est l'amidure de sodium.